# EUROPEAN PATENT SPECIFICATION

(11) **EP 2 268 301 B1**
(45) Date of publication and mention of the grant of the patent: **26.02.2020**
(21) Application number: 09703838.4
(22) Date of filing: 22.01.2009
(51) Int. Cl.: A61P 19/08, A61K 38/18, A61K 38/39, A61F 2/02

(54) **ERYTHROPOIETIN AND FIBRONECTIN COMPOSITIONS FOR BONE REGENERATION**
ERYTHROPOIETIN- UND FIBRONECTIN-ZUSAMMENSETZUNGEN ZUR KNOCHENNEUBILDUNG
COMPOSITIONS D'ÉRYTHROPOIÉTINE ET DE FIBRONECTINE POUR LA RÉGÉNÉRATION OSSEUSE

(30) Priority: 24.01.2008 US 6633
(43) Date of publication of application: 05.01.2011
(73) Proprietor: Remedor Biomed Ltd., 17111 Nazareth Ilit (IL)
(72) Inventor: HAMED, Saher, 17091 Nazareth Ilit (IL)
(74) Representative: Patentanwälte Bressel und Partner mbB
(86) International application number: PCT/IL2009/000087
(87) International publication number: WO 2009/093240

(56) References cited:
- EP-A- 1 550 715
- US-A1- 2002 160 033
- US-A1- 2005 147 645
- HOLSTEIN ET AL: "Erythropoietin (EPO) - EPO-receptor signaling improves early endochondral ossification and mechanical strength in fracture healing" LIFE SCIENCES, PERGAMON PRESS, OXFORD, GB, vol. 80, no. 10, 3 February 2007 (2007-02-03), pages 893-900, XP005873459 ISSN: 0024-3205 cited in the application

## Description

### FIELD AND BACKGROUND OF THE INVENTION

The present invention, in some embodiments thereof, relates to erythropoietin and fibronectin compositions and, more particularly, but not exclusively, to the use of same for bone regeneration.

Bone regeneration as well as fracture healing are complex processes requiring the collaborative efforts of many different cell types and factors. The contribution of each component is essential to reach optimization in these settings. Bone injuries, such as those resulting from diseases or fractures, need to be healed efficiently and more importantly, rapidly.

Bone is a dynamic living tissue and is continuously being replenished by resorption and deposition of bone matrix. This rigid tissue provides shape and support for the body, as well as protection for some organs. It also serves as a storage site for minerals and provides the medium - marrow - for the development and storage of blood cells. Because bones come in a variety of shapes and have a complex internal and external structure, they are lightweight, yet strong and hard.

One type of tissue that makes up bone is the mineralized osseous tissue (also called bone tissue) that gives it rigidity and honeycomb-like three-dimensional internal structure. Osseous tissue is a relatively hard and lightweight composite material formed mostly of calcium phosphate in the chemical arrangement termed calcium hydroxylapatite. All bones consist of living cells embedded in the mineralized organic matrix that makes up the osseous tissue. Other types of tissues found in bones include marrow, endosteum, periosteum, nerves, blood vessels and cartilage.

Bone is not a uniformly solid material, but rather has some spaces between its hard elements. The hard outer layer of bones is composed of compact bone tissue (also referred to as dense bone or cortical bone), so-called due to its minimal gaps and spaces, and thus gives bones their smooth, white, and solid appearance. Filling the interior of the organ is the trabecular bone tissue (an open cell porous network also called cancellous or spongy bone) which is comprised of a network of rod- and plate-like elements that make the overall organ lighter and allows space for blood vessels and marrow. Moreover, while bone is essentially brittle, it does have a significant degree of elasticity contributed chiefly by collagen.

Several types of cells comprise the bone including Osteoblasts, Osteocytes and Osteoclasts. Surrounding the cells is the matrix (the major constituent of bone) which comprises inorganic and organic parts. The inorganic part is mainly crystalline mineral, salts and calcium, which is present in the form of hydroxyapatite. The organic part is mainly Type I collagen. The organic part of the matrix also includes various growth factors, glycosaminoglycans, osteocalcin, osteonectin, bone sialo protein and Cell Attachment Factor.

One of the main things that distinguish the matrix of a bone from that of other cells is that the matrix in bone is hard.

The healing potential of bone, whether in osteopathy (e.x. osteoporosis, osteomamalcia, richets) or in a fracture or fusion model (e.g. bone transplantation) is influenced by a variety of biochemical, biomechanical, cellular, hormonal and pathological mechanisms. A continuously occurring state of bone deposition, resorption, and remodeling facilitates the healing process. For example, fracture healing, which restores the tissue to its original physical and mechanical properties, is influenced by a variety of systemic and local factors. Healing occurs in three distinct but overlapping stages: 1) the early inflammatory stage; 2) the repair stage; and 3) the late remodeling stage [Tsiridis E. (2007), Journal of Injury (38) 1:S11-25; Kalfas IH et al., Neurosurg Focus (2001) 15; 10(4):E1]. Because the functions of bone are numerous and complex, there are many disorders that require clinical care by a physician or other healthcare professional.

Various approaches for using Erythropoietin (EPO) or Fibronectin (FN) for bone regeneration have been attempted, some are summarized infra.

Administration of EPO for bone healing has been described by Holstein et al. in a murine closed femur fracture model [Holstein et al., Life sciences (2007) 80(10): 893-900], According to their teachings, daily i.p. injections of 5000 U/kg EPO from day 1 before fracture until day 4 after fracture resulted in an increased fraction of mineralized bone and osteoid in treated mice (EPO enhanced the transition of soft callus to hard callus).

U S. Pat. No. 7,473,678 discloses methods for promoting growth of bone, periodontium, ligament, or cartilage in a mammal by applying a composition comprising platelet-derived growth factor (PDGF). The composition may further comprise EPO.

U.S. Publication No. 20080031850 discloses the use of haematopoietic growth factors, in particular erythropoietin (EPO) and thrombopoietin (TPO), for promoting structural tissue regeneration (such as bone).

U.S. Pat. No. 5,264,214 discloses compositions for bone repair. The compositions described comprise collagen chemically conjugated to a synthetic hydrophilic polymer and may further comprise EPO. Moreover, the collagen may be crosslinked to proteins such as FN.

U.S. Publication No. 20050191248 teaches fibrosing agents for bone regeneration including FN and EPO.

U.S. Publication No. 20050147645 discloses a composition for modulating bone regeneration composition comprises a matrix selected from the group consisting of glycolic acid, lactic acid, collagen, demineralized bone, or a combination thereof. A first biologically active molecule comprising a fibronectin is attached to a portion of the matrix, to facilitate osteoblast activity and for promoting an increase in bone formation. A second biologically active molecule comprising a vitronectin, selected for its ability to attract osteoclasts and produce an inhibiting effect on osteoclast activity to thereby promote a decrease in bone resorption, is also attached to a portion of the matrix.

US 2002160033 A1 discloses a porous implantable medical device comprising fibronectin and erythropoietin, wherein the latter is expressed by cells bound to the implant.

### SUMMARY OF THE INVENTION

According to the present invention there is provided a composition according to claim 1. The invention provides a composition for use in treating a bone injury to promote bone regeneration in a subject in need thereof, wherein the composition comprises a therapeutically effective amount of Erythropoietin, Fibronectin, and platelet-derived growth factor (PDGF), wherein,
(i) said therapeutically effective amount of said Erythropoietin is selected from the group consisting of:
   about 1 - 50 µg/Kg for systemic administration; and
   about 0.1 - 50 µg/ml for local administration, and
(ii) said therapeutically effective amount of said Fibronectin is selected from the group consisting of:
   about 100 - 1000 µg/ml for systemic administration; and
   about 50- 500 µg/ml for local administration, and
(iii) said therapeutically effective amount of said platelet-derived growth factor is selected from the group consisting of:
   about 10 - 100 ng/ml for systemic administration; and
   about 1- 50 ng/ml for local administration.

The description also discloses a method of promoting bone regeneration in a subject in need thereof, the method comprising administering to the subject said composition.

The present description discloses an orthopedic graft comprising Erythropoietin and Fibronectin.

According to some embodiments of the invention, the systemic administration is selected from the group consisting of intravenous and intrabone infusion.

According to some embodiments of the invention, the local administration is selected from the group consisting of coated implant, coated synthetic bone and coated bone graft.

According to some aspects the administering may be effected at least once a day.

According to some embodiments of the invention, the composition further comprises at least one compound capable of promoting bone regeneration.

According to some embodiments of the invention, the at least one compound is selected from the group consisting of a bone morphogenic factor, a bone morphogenetic protein (BMP), an anti-resorptive agent, an osteogenic factor, a cartilage-derived morphogenetic protein, a parathyroid hormone, insulin-like growth factor-I (IGF-I), fibroblast growth factor (FGF), a transforming growth factor, a noggin, an osteogenic growth peptide, a growth hormone, an estrogen, a bisphosphonate, a statin, a calcitonin, a dihydroxy vitamin D3, a calcium preparation and a differentiating factor.

According to some embodiments of the invention, the BMP comprises BMP2, BMP4 and BMP7.

According to some embodiments of the invention, the subject has a medical condition selected from the group consisting of osteoporosis, bone fracture or deficiency, primary or secondary hyperparathyroidism, osteoarthritis, periodontal disease or defect, an osteolytic bone disease, post-plastic surgery, post-orthopedic implantation, and post-dental implantation.

According to some embodiments of the invention, the subject is a human being.

The administering of the Erythropoietin and the Fibronectin may be effected concomitantly.

According to some embodiments of the invention, the Erythropoietin the platelet-derived growth factor and the Fibronectin are in a co-formulation.

According to some embodiments of the invention, the Erythropoietin the platelet-derived growth factor and the Fibronectin are in separate formulations.

Unless otherwise defined, all technical and/or scientific terms used herein have the same meaning as commonly understood by one of ordinary skill in the art to which the invention.

### BRIEF DESCRIPTION OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:
FIG. 1 is a bar graph depicting the effects of erythropoietin (EPO) on osteoblast proliferation. Primary osteoblast cell cultures were treated with vehicle (left column), 2 µg/ml recombinant EPO (middle column) or 10 µg/ml recombinant EPO (right column) in serum-free media for 24 hours, followed by 60-minute incubation at 37 °C with 10µM BrdU. BrdU⁺ cells were detected using a mouse monoclonal antibody specific to BrdU.

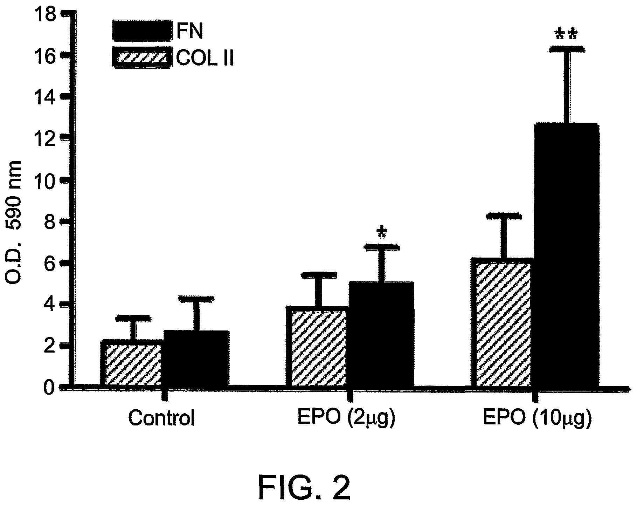

FIG. 2 is a bar graph depicting the effect of EPO and Fibronectin (FN) or collagen type Il on osteoblast adhesion. 96-well tissue culture plates were coated with 100 µg/ml FN (black columns) or 125 µg/ml collagen type II (slanted line columns). Osteoblasts, treated with vehicle (left column), 2 µg/ml recombinant EPO (middle column) or 10 µg/ml recombinant EPO (right column) for 1 hour in serum-free media, were allowed to attach to the tissue culture plates for 1 hour at 37 °C. Adherent cells were fixed, stained, and examined at OD 590 nm. Each assay was carried out in six separate wells and was repeated in three independent experiments.
FIGs. 3A-B are bar graphs depicting the effect of EPO on the proliferation of primary human osteoblasts (HOB). Figure 3A shows proliferation of HOBs with EPO alone; and Figure 3B shows proliferation of HOBs with the combination of EPO and FN.
FIG. 4 is a bar graph depicting the effect of EPO, PDGF or both (all with FN) on proliferation of HOBs.
FIGs. 5A-B are bar graphs depicting the effect of EPO on osteocalcin and BMPs expression on HOBs. Figure 5A shows the effect of EPO alone on osteocalcin and BMPs expression; and Figure 5B shows the effect of the combination of EPO and FN on osteocalcin and BMPs expression.

### DESCRIPTION OF SPECIFIC EMBODIMENTS OF THE INVENTION

The principles and operation of the present invention may be better understood with reference to the drawings and accompanying descriptions.

While reducing the present invention to practice, the present inventor has uncovered that specific concentrations of Erythropoietin (EPO), Fibronectin (FN) and platelet-derived growth factor (PDGF) significantly accelerate osteoblast proliferation, adhesion and expression of Bone Morphogenetic Proteins (BMPs) and Osteocalcin therein. These findings suggest the use of the present teachings in promoting bone regeneration.

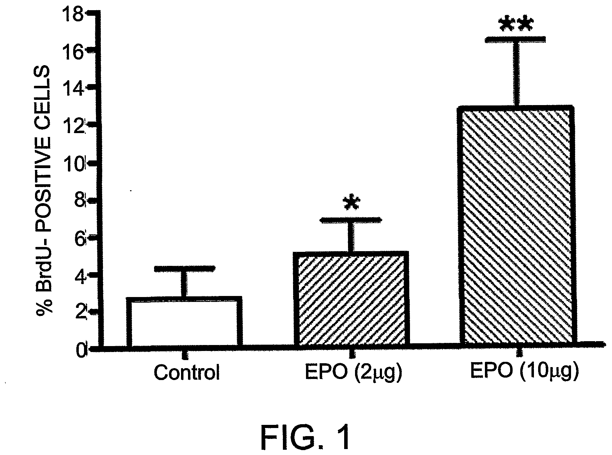

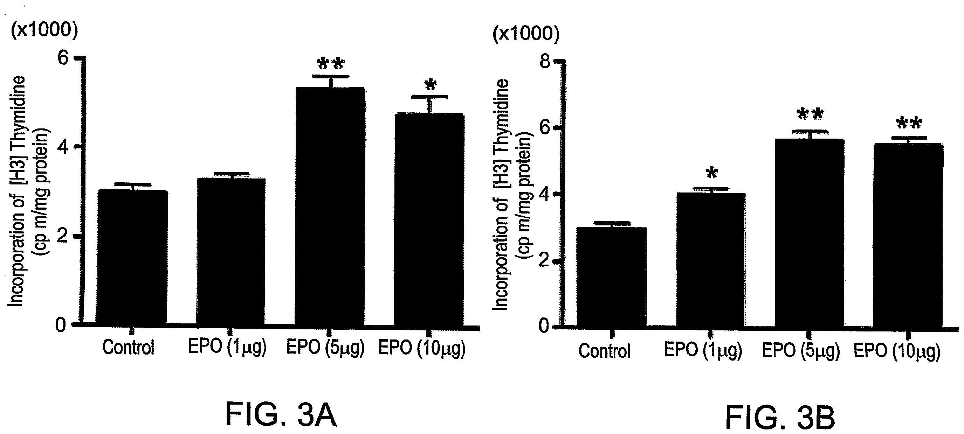

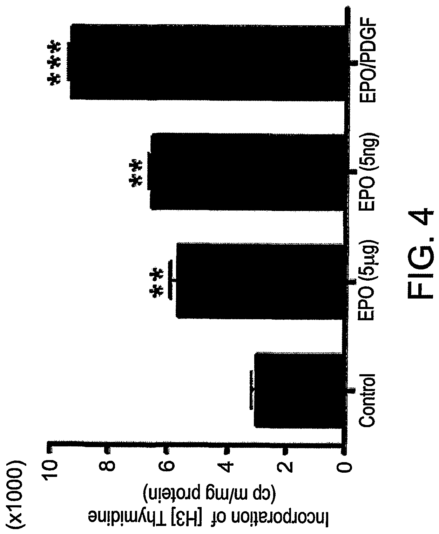

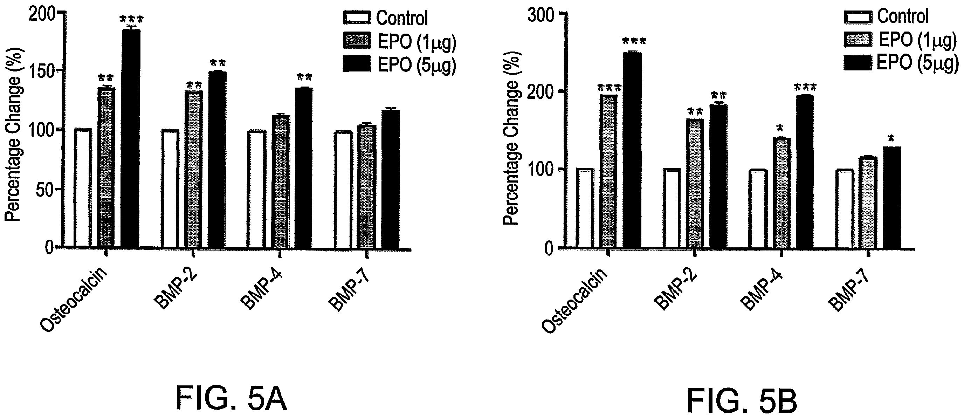

As is shown hereinbelow and in the Examples section which follows, the present inventor has uncovered through laborious experimentation that certain concentrations of EPO, FN and PDGF are desirable for osteogenesis. The present inventor has specifically shown that EPO promotes osteoblast proliferation and cell adhesion in a dose dependent manner (Figures 1-2). Moreover, growth of osteoblasts in the presence of both EPO and FN results in significantly higher adhesion levels (Figure 2), proliferation levels (Figures 3A-B) and expression of BMPs and Osteocalcin (Figures 5A-B) by theses bone cells. In addition, as is shown in Figure 4, the addition of PDGF to EPO and FN resulted in a considerably enhanced proliferation of these cells. Taken together, these results substantiate the value of EPO, FN and PDGF in promoting bone regeneration.

Thus, the present description discloses a method of promoting bone regeneration in a subject in need thereof, the method comprising administering to the subject a therapeutically effective amount of Erythropoietin, Fibronectin and platelet-derived growth factor, thereby promoting bone regeneration in the subject, wherein the therapeutically effective amount of the Erythropoietin is selected from the group consisting of about 1 - 50 µg/Kg for systemic administration; and about 0.1 - 50 µg/ml for local administration, and wherein the therapeutically effective amount of the Fibronectin is selected from the group consisting of about 100 - 1000 µg/ml for systemic administration; and about 50 - 500 µg/ml for local administration and wherein said therapeutically effective amount of said platelet-derived growth factor is selected from the group consisting of about 10 - 100 ng/ml for systemic administration; and about 1- 50 ng/ml for local administration.

The term "bone regeneration" as used herein refers to promoting bone formation (e.g., by osteoblast prioliferation) and optionally inhibiting bone resorption.

The term "promoting" in respect to bone regeneration refers to the process of increasing the amount of bone tissue, bone cells, bone cell differentiation, bone matrix, etc. in a manner that allows bone regeneration. Thus in some embodiments of the present invention, promoting refers to at least about 10 %, 20 %, 50 %, 80 %, 100 % or more increase in bone regeneration or at least about 10 %, 20 %, 50 %, 80 % arrest in bone resorption. Those of skill in the art will understand that various methodologies and assays can be used to assess the promotion of bone regeneration, and similarly, various methodologies and assays may be used to assess the arrest of bone degradation or resorption.

As used herein, the phrase "bone tissue" refers to the osseous tissue which makes up the rigid part of the bone organs. The term bone tissue refers to both spongy and compact osseous tissues.

It will be appreciated that any type of bone may be regenerated according to the present teachings including long bones (e.g. bones of the limbs, including those of the fingers and toes), short bones (e.g. bones of the wrist and ankle), flat bones (e.g. bones of the skull, sternum), irregular bones (e.g. bones of the spine and hips) and sesamoid bones (e.g. patella and the pisiform bones).

As used herein, the phrase "bone cell" refers to a skeletal tissue cell, such as, bone, cartilage, tendon, ligament, marrow stroma and connective tissue cells, including bone forming cells such as osteoblasts, bone resorbing cells such as osteoclasts, macrophages, scavenger cells and progenitor bone cells such as stromal, osteogenic cell or a bone resorbing cell progenitor.

As used herein, the phrase "bone matrix" refers to the intercellular substance of the bone tissue consisting of inorganic material such as crystalline mineral, salts calcium and hydroxylapatite, and organic material such as collagen (e.g. Type I collagen), growth factors, glycosaminoglycans, osteocalcin, osteonectin, bone sialo protein and Cell Attachment Factor.

Compositions of the present invention are envisioned to promote bone regeneration in a subject of need thereof.

As used herein the term "subject" refers to any mammal, such as a human subject or a domesticated animal including, but not limited to, horses (i.e. equine), cattle, goat, sheep, pig, dog, cat, camel, alpaca, llama and yak, male or female at any age that experiences or may experience bone damage, at any stage and/or degree.

It will be appreciated that the compositions for use according to the present invention can be used for treating or preventing any bone deficit-related disease or condition such as, but not limited to, preventing bone defects and deficiencies in closed, open and non-union fractures; augmenting bone mass in young individuals at risk; prophylactic treatment in young individuals by enhancing peak bone mass in closed and open fracture reduction; promotion of bone healing in plastic surgery or post-plastic surgery; stimulation of bone ingrowth into non-cemented post orthopedic and dental implants; elevation of peak bone mass in pre-menopausal women; treatment of growth deficiencies; treatment of primary or secondary hyperparathyroidism; treatment of osteolytic bone disease such as cancer; treatment of periodontal disease and defects, and other tooth repair processes; increase in bone formation during distraction osteogenesis; and treatment of other skeletal disorders, such as age-related osteoporosis, post-menopausal osteoporosis, glucocorticoid-induced osteoporosis or disuse osteoporosis and arthritis, osteoarthritis or any condition that benefits from stimulation of bone formation, on the one hand, and inhibition of bone resorption, on the other. The agents of the composition can also be useful in repair of congenital, trauma-induced or surgical resection of bone (for instance, for cancer treatment), and in cosmetic surgery. Further, the compositions for use according to the present invention can be used for limiting or treating cartilage defects or disorders, and may be useful in wound healing or tissue repair in which bone has been damaged.

Additional bone related diseases or conditions which may be treated according to the present teachings include Bone cyst, Bone spur (Osteophytes), Bone tumor, Giant cell tumor of bone, Osteosarcoma, Craniosynostosis, Fibrodysplasia ossificans progressive, Fibrous dysplasia, Hypophosphatasia, Klippel-Feil syndrome, Metabolic Bone Disease, Osteitis deformans (or Paget's disease of bone), Osteitis fibrosa cystica (or Osteitis fibrosa, or Von Recklinghausen's disease of bone), Osteitis pubis, Condensing osteitis (or Osteitis condensans), Osteitis condensans ilii, Osteochondritis dissecans, Osteochondroma (Bone Tumor), Osteogenesis Imperfecta, Osteomalcia, Osteomyelitis, Osteopenia, Osteopetrosis, Porotic hyperostosis and Renal Osteodystrophy.

The phrase "treating or preventing" as used herein refers to a postponement of development of bone deficit symptoms and/or a reduction in the severity of such symptoms that will or are expected to develop. These further include ameliorating existing bone or cartilage deficit symptoms, preventing additional symptoms, ameliorating or preventing the underlying metabolic causes of symptoms, preventing or reversing bone resorption and/or encouraging bone growth. Those of skill in the art will understand that various methodologies and assays can be used to assess the development of a condition, and similarly, various methodologies and assays may be used to assess the reduction, remission or regression of the condition.

Treatment can be evaluated by routine experimentation, using models which are well known in the art (e.g. murine and canine animal models). Additional parameters known in the art can be quantified for determining bone regeneration in a subject, such as by bone X-ray.

As used herein the term "Erythropoietin" refers to a mammalian (e.g., human) Erythropoietin protein (interchangeably used with polypeptide) or mimetics thereof such as set forth in GenBank Accession No. NP_000790. Erythropoietin may be synthesized using recombinant DNA techniques or solid phase technology. Erythropoietin is also commercially available (e.g., Cytolab/Peprotech, Rehovot, Israel; Arenesp, Amgen, Thousand Oaks, CA, USA; and Epogen, Amgen, Thousand Oaks, CA, USA, Bristol-Myers Squibb, Roche and Sanofi-Aventis). Erythropoietin may be used as an entire glycoprotein or as only a protein subunit devoid of the bound sugar. Since the Erythropoietin is used here for clinical applications, it is preferably sterile or may be purified of possible contaminating factors (e.g., bacteria or bacterial components, such as by filter).

As used herein the term "Fibronectin" refers to a mammalian (e.g., human) Fibronectin protein (interchangeably used with polypeptide) or mimetics thereof such as set forth in GenBank Accession No. NP_002017. Fibronectin may be synthesized using recombinant DNA techniques or solid phase technology. Fibronectin is also commercially available (e.g., Chemicon International Inc., Temecula, CA, USA). Since the Fibronectin is used here for clinical applications, it is preferably sterile or may be purified of possible contaminating factors (e.g., bacteria or bacterial components, such as by filter).

It will be appreciated that when mimetics compositions are used the dosages of FN and EPO should be calibrated such as according to the molar value. Such a calibration is a routine calculation for those of ordinary skill in the art. Composition for use according to the present invention may comprise Erythropoietin, Fibronectin in a co-formulation or in two separate compositions. It will be appreciated, that when not co-formulated, administration of Erythropoietin and Fibronectin may be effected concomitantly or sequentially.

It will be appreciated, that according to some embodiments of the present teachings, Erythropoietin and Fibronectin may be administered via a systemic administration or via a local administration.

As used herein the phrase "systemic administration" refers to oral, intravenous, intraperitoneal and intramuscular administration of the compositions for use according to the present invention.

According to an exemplary embodiment, the compositions for use according to the present invention are effected intravenously. According to another exemplary embodiment, the compositions of the present invention are directly administered to the damaged bone via intrabone infusion.

As used herein the phrase "local administering" refers to applying the compositions of the present invention in close proximity to the damaged bone.

The composition for use according to the present invention may also be delivered directly to the bone in need of regeneration by coating or embedding the compositions on bone implants (e.g. drug eluting implants), on synthetic bone (e.g. drug eluting substitutes), on bone graft, on orthopedic coated implants including bone screws, titanium implants or on other fixation devices. The compositions for use according to the present invention may be coated or embedded within the implanted devices using any method known to one of ordinary skill in the art, as for example by placing the implant in solution containing the therapeutic agent between film growth cycles or by dissolving the desired agent into the supersaturated solution and adsorbing into a calcium phosphate coating of the implant [see for example, Development in new materials - Journal Materials Research Innovations (1999) Springer Berlin / Heidelberg Publishers, Volume 3(3): 179-180; and Pioletti et al., Source: Current Drug Delivery (2008), Volume 5(1), pp. 59].

The configurations and shapes of the implant for use according to the present invention are not particularly limited. The implant can take any desired configurations or shapes, such as sponges, meshes, unwoven fabric products, discs, films, sticks, particles, or pastes. The implant for use according to the present invention may be used in combination with other materials. For example, the growth factors can be mixed with hydroxyapatite granules, and the resultant can be used as a bone filler. These configurations and shapes may be suitably selected depending on the applications of the implant.

It will be appreciated that the dose of Erythropoietin and Fibronectin applied according to the teachings of the present invention may vary. Thus, Erythropoietin can be administered at a dose between 1 - 50 µg/Kg for systemic administration or at a dose between 0.1 - 50 µg/ml for local administration depending on the severity of the bone damage to be treated. Fibronectin can be administered at a dose between 100 - 1000 µg/ml for systemic administration or at a dose between 50 - 500 µg/ml for local administration depending on the severity of the bone damage to be treated.

As used herein the term "platelet-derived growth factor" refers to a mammalian (e.g., human) PDGF protein (interchangeably used with polypeptide) or mimetics thereof such as set forth in GenBank Accession Nos. NP_148983, NP_002598. PDGF may be synthesized using recombinant DNA techniques or solid phase technology. PDGF is also commercially available (e.g., Sigma). Since the PDGF is used here for clinical applications, it is preferably sterile or may be purified of possible contaminating factors (e.g., bacteria or bacterial components, such as by filter).

It will be appreciated that PDGF may be formulated in a co-formulation or in separate compositions with respect to Erythropoietin and Fibronectin. It will be appreciated, that when not co-formulated, administration of PDGF may be effected concomitantly or sequentially to Erythropoietin and Fibronectin.

According to the present teachings, the dose of PDGF administered to the subject may vary. Thus, PDGF can be administered at a dose between 10-100 ng/ml for systemic administration or at a dose between 1 - 50 ng/ml for local administration.

The compositions for use according to the present invention can be administered to the subject *per se* or in a pharmaceutical composition.

As used herein a "pharmaceutical composition" refers to a preparation of the active ingredients described herein with other chemical components such as physiologically suitable carriers and excipients. The purpose of the composition is to facilitate administration of the active ingredients (EPO, FN, PDGF) to the subject.

As used herein the term "active ingredient" refers to the Erythropoietin, Fibronectin and PDGF compositions accountable for the intended biological effect (i.e., promoting bone regeneration).

Hereinafter, the phrases "physiologically acceptable carrier" and "pharmaceutically acceptable carrier" which may be interchangeably used refer to a carrier or a diluent that does not cause significant irritation to the subject and does not abrogate the biological activity and properties of the administered active ingredients. An adjuvant is included under these phrases.

Herein, the term "excipient" refers to an inert substance added to the composition 10 (pharmaceutical composition) to further facilitate administration of an active ingredient of the present invention.

It will be appreciated that the pharmaceutical composition for use according to the present invention may also include one or more compounds which promotes bone formation and/or inhibits bone resorption, such as, for example, bone morphogenic factors, bone morphogenic proteins including BMP1, BMP2, BMP3, BMP4, BMP5, BMP6, BMP7, BMP8a, BMP8b, BMP10 and BMP15, parathyroid hormones, noggin, osteogenic growth peptides, anti-resorptive agents, osteogenic factors, cartilage-derived morphogenic proteins, growth hormones, cytokines such as fibroblast growth factor (FGF), insulin-like growth factor-I (IGF-I), transforming growth factors, estrogens, bisphosphonates, statin, calcitonin, dihydroxy vitamin D₃, and calcium preparations are preferred for this purpose.

Techniques for formulation and administration of drugs may be found in "Remington's Pharmaceutical Sciences," Mack Publishing Co., Easton, PA, latest edition, which is incorporated herein by reference.

As mentioned hereinabove, suitable routes of administration may, for example, include a systemic manner including oral, rectal, transmucosal, especially transnasal, intestinal or parenteral delivery, including intramuscular, subcutaneous and intramedullary injections as well as intrathecal, direct intraventricular, intravenous, inrtaperitoneal, intramuscular, intranasal, or intraocular injections. It will be appreciated that the compositions for use according to the present invention may also be administered via intrabone infusions.

Alternately, one may administer the pharmaceutical composition in a local rather than systemic manner, for example, via injection of the pharmaceutical composition directly into a bone tissue region of a patient, or via application of the compositions directly into a tissue region in proximity to the damaged bone of a patient. Suitable routes of administration of the compositions may, for example, include topical (e.g., to a keratinous tissue, such as the skin, scalp) and mucosal (e.g., oral, vaginal, eye) administrations.

Pharmaceutical compositions for use according to the present invention may be manufactured by processes well known in the art, e.g., by means of conventional mixing, dissolving, granulating, dragee-making, levigating, emulsifying, encapsulating, entrapping or lyophilizing processes.

Pharmaceutical compositions for use in accordance with the present invention thus may be formulated in conventional manner using one or more physiologically acceptable carriers comprising excipients and auxiliaries, which facilitate processing of the active ingredients into preparations which, can be used pharmaceutically. Proper formulation is dependent upon the route of administration chosen.

For injection, the active ingredients of the pharmaceutical composition may be formulated in aqueous solutions, preferably in physiologically compatible buffers such as Hank's solution, Ringer's solution, or physiological salt buffer. For transmucosal administration, penetrants appropriate to the barrier to be permeated are used in the 10 formulation. Such penetrants are generally known in the art.

For oral administration, the pharmaceutical composition can be formulated readily by combining the active compounds with pharmaceutically acceptable carriers well known in the art. Such carriers enable the pharmaceutical composition to be formulated as tablets, pills, dragees, capsules, liquids, gels, syrups, slurries, suspensions, and the like, for oral ingestion by a patient. Pharmacological preparations for oral use can be made using a solid excipient, optionally grinding the resulting mixture, and processing the mixture of granules, after adding suitable auxiliaries if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carbomethylcellulose; and/or physiologically acceptable polymers such as polyvinylpyrrolidone (PVP). If desired, disintegrating agents may be added, such as crosslinked polyvinyl pyrrolidone, agar, or alginic acid or a salt thereof such as sodium alginate.

Dragee cores are provided with suitable coatings. For this purpose, concentrated sugar solutions may be used which may optionally contain gum arabic, talc, polyvinyl pyrrolidone, carbopol gel, polyethylene glycol, titanium dioxide, lacquer solutions and suitable organic solvents or solvent mixtures. Dyestuffs or pigments may be added to the tablets or dragee coatings for identification or to characterize different combinations of active compound doses.

Pharmaceutical compositions which can be used orally, include push-fit capsules made of gelatin as well as soft, sealed capsules made of gelatin and a plasticizer, such as glycerol or sorbitol. The push-fit capsules may contain the active ingredients in admixture with filler such as lactose, binders such as starches, lubricants such as talc or magnesium stearate and, optionally, stabilizers. In soft capsules, the active ingredients may be dissolved or suspended in suitable liquids, such as fatty oils, liquid paraffin, or liquid polyethylene glycols. In addition, stabilizers may be added. All formulations for oral administration should be in dosages suitable for the chosen route of administration.

For buccal administration, the compositions may take the form of tablets or lozenges formulated in conventional manner.

For administration by nasal inhalation, the active ingredients for use according to the present invention are conveniently delivered in the form of an aerosol spray presentation from a pressurized pack or a nebulizer with the use of a suitable propellant, e.g., dichlorodifluoromethane, trichlorofluoromethane, dichloro-tetrafluoroethane or carbon dioxide. In the case of a pressurized aerosol, the dosage unit may be determined by providing a valve to deliver a metered amount. Capsules and cartridges of, e.g., gelatin for use in a dispenser may be formulated containing a powder mix of the compound and a suitable powder base such as lactose or starch.

The pharmaceutical composition described herein may be formulated for parenteral administration, e.g., by bolus injection or continuos infusion. Formulations for injection may be presented in unit dosage form, e.g., in ampoules or in multidose containers with optionally, an added preservative. The compositions may be suspensions, solutions or emulsions in oily or aqueous vehicles, and may contain formulatory agents such as suspending, stabilizing and/or dispersing agents.

Pharmaceutical compositions for parenteral administration include aqueous solutions of the active preparation in water-soluble form. Additionally, suspensions of the active ingredients may be prepared as appropriate oily or water based injection suspensions. Suitable lipophilic solvents or vehicles include fatty oils such as sesame oil, or synthetic fatty acids esters such as ethyl oleate, triglycerides or liposomes. Aqueous injection suspensions may contain substances, which increase the viscosity of the suspension, such as sodium carboxymethyl cellulose, sorbitol or dextran. Optionally, the suspension may also contain suitable stabilizers or agents which increase the solubility of the active ingredients to allow for the preparation of highly concentrated solutions.

Alternatively, the active ingredient may be in powder form for constitution with a suitable vehicle, e.g., sterile, pyrogen-free water based solution, before use.

The pharmaceutical composition of the present invention may also be formulated in rectal compositions such as suppositories or retention enemas, using, e.g., conventional suppository bases such as cocoa butter or other glycerides.

Pharmaceutical compositions suitable for use in context of the present invention include compositions wherein the active ingredients are contained in an amount effective to achieve the intended purpose. More specifically, a therapeutically effective amount means an amount of active ingredients (EPO, FN, PDGF) effective to prevent, alleviate or ameliorate symptoms of a disorder (e.g., bone damange) or prolong the survival of the subject being treated.

Determination of a therapeutically effective amount is well within the capability of those skilled in the art, especially in light of the detailed disclosure provided herein.

For any preparation used in the methods of the invention, the therapeutically effective amount or dose can be estimated initially from in vitro and cell culture assays. For example, a dose can be formulated in animal models to achieve a desired concentration or titer. Such information can be used to more accurately determine useful doses in humans.

Toxicity and therapeutic efficacy of the active ingredients described herein can be determined by standard pharmaceutical procedures in vitro, in cell cultures or experimental animals. The data obtained from these in vitro and cell culture assays and animal studies can be used in formulating a range of dosage for use in human. The dosage may vary depending upon the dosage form employed and the route of administration utilized. The exact formulation, route of administration and dosage can be chosen by the individual physician in view of the patient's condition. (See e.g., Fingl, et al., 1975, in "The Pharmacological Basis of Therapeutics", Ch. 1 p.1).

Dosage amount and interval may be adjusted individually to levels of the active ingredient which are sufficient to induce or suppress the biological effect (minimal effective concentration, MEC). The MEC will vary for each preparation, but can be estimated from in vitro data. Dosages necessary to achieve the MEC will depend on individual characteristics and route of administration. Detection assays can be used to determine plasma concentrations.

An animal model which can be used according to the present teachings to assess the biological effect of the compositions described herein includes an animal model for long bone fractures of Female Sprague-Dawley rats. This model introduces a critical size tibia defect into the rats.

Depending on the severity of the condition (e.g., the area, depth and degree of the bone damage) and the responsiveness of the tissue, dosing can be of a single or a plurality of administrations, with course of treatment lasting from several days to several weeks or months or until cure is effected or until ample bone has been regenerated. Preferably, the compositions for use according to the present invention are administered at least once a day.

The amount of a composition to be administered will, of course, be dependent on the subject being treated, the severity of the affliction, the manner of administration, the judgment of the prescribing physician, etc.

Compositions for use according to the present invention may, if desired, be presented in a pack or dispenser device, such as an FDA-approved kit, which may contain one or more unit dosage forms containing the active ingredient. The pack may, for example, comprise metal or plastic foil, such as a blister pack. The pack or dispenser device may be accompanied by instructions for administration. The pack or dispenser device may also be accompanied by a notice in a form prescribed by a governmental agency regulating the manufacture, use, or sale of pharmaceuticals, which notice is reflective of approval by the agency of the form of the compositions for human or veterinary administration. Such notice, for example, may include labeling approved by the U.S. Food and Drug Administration for prescription drugs or of an approved product insert. Compositions comprising a preparation of the invention formulated in a pharmaceutically acceptable carrier may also be prepared, placed in an appropriate container, and labeled for treatment of an indicated condition, as further detailed above.

Since the compositions for use according to the present invention are utilized *in vivo,* the compositions are preferably of high purity and substantially free of potentially harmful contaminants, e.g., at least National Food (NF) grade, generally at least analytical grade, and preferably at least pharmaceutical grade. To the extent that a given compound must be synthesized prior to use, such synthesis or subsequent purification shall preferably result in a product that is substantially free of any potentially contaminating toxic agents that may have been used during the synthesis or purification procedures.

Additional factors may be incorporated into the compositions for use according to the present invention (i.e., Erythropoietin, Fibronectin and PDGF described hereinabove). These include, but are not limited to, extracellular matrix components (e.g. vitronectin, laminin, collagen, elastin), growth factors (e.g. FGF 1, FGF 2, IGF 1, IGF 2, PDGF, EGF, KGF, HGF, VEGF, SDF-1, GM-CSF, CSF, G-CSF, TGF alpha, TGF beta, NGF and ECGF), hypoxia inducible factors (e.g. HIF-1 alpha and beta and HIF-2), hormones (e.g., insulin, growth hormone (GH), CRH, Leptin, Prolactin and TSH), angiogenic factors (e.g., angiogenin and angiopoietin), coagulation and anticoagulation factors [e.g., Factor I, Factor XIII, tissue factor, calcium, vWF, protein C, protein S, protein Z, fibronectin, antithrombin, heparin, plasminogen, low molecular weight heparin (Clixan), high molecular weight kininogen (HMWK), prekallikrein, plasminogen activator inhibitor-1 (PAI1), plasminogen activator inhibitor-2 (PAI2), urokinase, thrombomoduline, tissue plasminogen activator (tPA), alpha 2-antiplasmin and Protein Z-related protease inhibitor (ZPI)], cytokines (IL-1 alpha, IL-1 beta, IL-2, IL-3, IL-4, IL-5, IL-6, IL-7, IL-8, IL-9, IL-10, IL-11, IL-12, IL-13 and INF-alpha, INF, beta, and INF-gamma), chemokines (e.g., MCP-1 or CCL2), enzymes (e.g. endoglycosidases, exoglycosidases, endonucleases, exonucleases, peptidases, lipases, oxidases, decarboxylases, hydrases, chondroitinase, chondroitinase ABC, chondroitinase AC, hyaluronidase, keratanase, heparanases, heparanase splice variance, collagenase, trypsin, catalases), neurotransmitters (e.g., acetylcholine and monoamines), neuropeptides (e.g. substance P), vitamins (e.g., D-biotin, Choline Chloride, Folic acid, Myo-inositol, Niacinamide, D-Pantothenic acid, Calcium salts, Pyridoxal.HCI, Pyrodixine.HCI, Riboflavin, Thiamine.HCI, Vitamin B12, vitamin E, vitamin C, vitamin D, vitamin B1-6, vitamin K, vitamin A and vitamin PP), carbohydrates (e.g. Mono/Di/Polysacharides including glucose, mannose, maltose and fructose), ions, chelators (e.g. Fe chelators, Ca chelators), antioxidants (e.g., Vitamin E, Quarcetin, superoxide scavengers, Superoxide dismutase), H2O2 scavengers, free radicals scavengers, Fe scavengers), fatty acids (e.g., Triglycerides, Phospholipids, Cholesterols, free fatty acids and non free fatty acids, fatty alcohol, Linoleic acid, oleic acid and lipoic acid), antibiotics (e.g., Penicillins, Cephalosporins and Tetracyclines), analgesics, anesthetics, antibacterial agents, anti-yeast agents, anti-fungal agents, antiviral agents, pro-biotic agents, anti-protozal agents, anti-pruritic agents, anti-dermatitis agents, anti-emetics, anti-inflammatory agents, anti-hyperkeratolyic agents, antiperspirants, anti-psoriatic agents, anti-seborrheic agents, antihistamine agents, amino acids (e.g., essential and non essential (from A-Z) especially glutamine and arginine), salts (e.g., prurivat salts and sulfate salts), sulfates (e.g. Calcium Sulfate), steroids (e.g., androgens, estrogens, progestagens, glucocorticoids and mineralocorticoids), catecholamines (e.g., Epinephrine and Nor-epinephrine), Nucleosides and Nucleotides (e.g., Purins and Pyrimidines), Prostaglandins (e.g. Prostaglandin E2), Leucotriens, Erythropoietins (e.g. Thrombopoietin), Proteoglycans (e.g. Heparan sulfate, keratan sulfate), Hydroxyapatites [e.g. Hydroxyapatite (Ca₁₀(PO₄)₆(OH)₂)], Haptoglobins (Hp1-1, Hp2-2 and Hp1-2), Superoxide dismutases (e.g. SOD 1/2/3), Nitric Oxides, Nitric Oxide donors (e.g. nitroprusside, Sigma Aldrich, St. Louis, MO, USA, Glutathione peroxidases, Hydrating compounds (e.g. vasopressin), cells (e.g. Platelets), cell medium (e.g. M199, DMEM/F12, RPMI, Iscovs), serum (e.g. human serum, fetal calf serum, , fetal bovine serum), buffers (e.g., HEPES, Sodium Bicarbonate), detergents (e.g., Tween), disinfectants, herbs, fruit extracts, vegetable extracts (e.g. cabbage, cucumber), flower extracts, plant extracts, flavinoids (e.g. pomegranate juice), spices, leafs (e.g. Green tea, Chamomile), Polyphenols (e.g. Red Wine), honey, lectins, microparticles, nanoparticles (lyposomes), micelles, calcium carbonate (CaCO3, e.g. precipitated calcium carbonate, ground/pulverized calcium carbonate, albacar, PCC, GCC), calcite, limestone, crushed marble, ground limestone, lime, chalk (e.g. whiting chalk, champagne chalk, french chalk) and co factors such as BH4 (tetrahydrobiobterine).

The present composition may also contain ingredients, substances, elements and materials containing, hydrogen, alkyl groups, aryl groups, halo groups, hydroxy groups, alkoxy groups, alkylamino groups, dialkylamino groups, acyl groups, carboxyl groups, carboamido groups, sulfonamide groups, aminoacyl groups, amide groups, amine groups, nitro groups, organo selenium compounds, hydrocarbons, and cyclic hydrocarbons.

The present composition may be combined with substances such as benzol peroxide, vasoconstrictors, vasodilatators, salicylic acid, retinoic acid, azelaic acid, lactic acid, glycolic acid, pyreuric acid, tannins, benzlidenecamphor and derivatives thereof, alpha hydroxyis, surfactants.

Compositions for use according to some embodiments of the present invention may be bioconjugated to polyethylenglycol (e.g. PEG, SE-PEG) which preserves the stability (e.g., against protease activities) and/or solubility (e.g., within a biological fluid such as blood, digestive fluid) of the active ingredients (e.g. EPO, FN, PDGF compositions of the present invention) while preserving their biological activity and prolonging its half-life.

It will be appreciated that compositions for use according to the present invention can be used in combination with other currently practiced therapies for bone healing such as, without being limited to, low-intensity pulsed ultrasound, high-energy extracorporeal shock wave therapy (ESWT).

The present description also discloses an orthopedic graft comprising Erythropoietin and Fibronectin.

As used herein the term "orthopedic graft" refers to any allograft materials, xenograft materials, and synthetic materials which may be used for bone implantation.

An "allograft" as used herein refers to a graft of tissue, such as bone tissue, from a donor of one species and grafted into a recipient of the same species. Allograft tissue is typically derived from cadaveric donors (i. e. from deceased donors).

A "xenograft" as used herein refers to a graft of tissue, such as bone tissue, from a donor of one species and grafted into a recipient of another species (e.g. a porcine bone grafted into a human).

A "synthetic material" as used herein refers to an artificial graft material such as for example, ceramic graft materials such as calcium-based materials, calcium-phosphate-based materials, calcium-sulfate-based materials, calcium-sodium-phosphate-based materials, etc.

It will be appreciated that compositions for use according to the present invention comprising Erythropoietin, Fibronectin and PDGF may be embedded within the orthopedic graft or may be coated on the graft as described in detail hereinabove.

The terms "comprises", "comprising", "includes", "including", "having" and their conjugates mean "including but not limited to".

The term "consisting of means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format.

Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

Various embodiments and aspects of the present invention as delineated hereinabove and as claimed in the claims section below find experimental support in the following examples.

### EXAMPLES

Generally, the nomenclature used herein and the laboratory procedures utilized in the present description include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., Eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996); all of which are incorporated by reference as if fully set forth herein. Other general references are provided throughout this document. The procedures therein are believed to be well known in the art and are provided for the convenience of the reader.

### EXAMPLE 1

### Enhanced in vitro proliferation and adhesion of osteoblasts by erythropoietin and fibronectin

### MATERIALS AND EXPERIMENTAL PROCEDURES

### Osteoblast-enriched cell isolation

Fetal rat calvarias (FRC) cell cultures were established using a modification of previously described techniques [Aronow MA. J Cell Physiol. (1990), 143(2):213-21], Briefly, frontal and parietal bones from Sprague-Dawley fetal rats at gestational day 21 were stripped of their periosteum and dura mater, minced into 1-mm fragments, and washed with sterile PBS. Calvarias were then serially digested with a solution of collagenase/dispase (Biological Industries) in a shaking incubator at 37 °C for 4 cycles of 10 minutes each. Fractions from cycles 2-5 were collected, centrifuged, and resuspended in culture media. Cells were plated in 75 mm culture dishes and allowed to grow to sub-confluence. To confirm isolation of osteoblast-enriched cultures, the ability of isolated cells to form mineralized bone nodules and produce alkaline phosphatase was assessed using standard techniques.

### Cell culture

The primary osteoblast cell cultures were grown in culture medium comprising DMEM supplemented with 10 % fetal bovine serum (Biological Industries), 100 IU/ml penicillin (Biological Industries), 50 µg/ml streptomycin (Biological Industries) and 100 µg/ml amphotericin B (Biological Industries). Media was changed every 2 days and cells were 40 passaged after trypsinization (0.05 % trypsin-EDTA, Biological Industries). The cells were then grown in a humidified tissue incubator at 5 % CO₂-95 % nitrogen gas mixture. In addition, a pH meter was used to analyze and monitor the media pH. All experiments were thus carried out at nearly constant neutral pH of 7.4-7.5.

### In vitro assays

To examine the effects of erythropoietin (EPO) on osteoblast proliferation, cells were treated with 2 µg/ml or 10 µg/ml recombinant EPO (Aranesp) or vehicle in serum-free media for 24 hours, followed by a 60 minute incubation at 37 °C with 10 µM BrdU (BD Pharmingen). BrdU⁺ cells were subsequently detected using a mouse monoclonal antibody to BrdU as described by the manufacturer's protocol (BD Pharmingen).

In addition, the DNA content of EPO or control treated cells was examined by flow cytometry. In short, cells were trypsinized, digested with RNase A for 30 minutes at 37 °C, stained with propidium iodide for 30 minutes at room temperature, and analyzed with a FACS calibur machine (BD Biosciences, Palo Alto, CA, USA).

For the cell attachment assays, 96-well plates were first coated with 100 µg/ml Fibronectin (FN, Chemicon Int.) or 125 µg/ml collagen type II (Sigma). Cells that had been treated with 2 µg/ml or 10 µg/ml recombinant EPO or vehicle for 1 hour in serum-free media were allowed to attach for 12 hours at 37 °C on the FN or Collagen-coated plates. Adherent cells were fixed, stained, and examined at OD 590 nm, as previously described [Kawaguchi N., J Cell Sci. (2003) 116(Pt 19):3893-904. Each assay was carried out in six separate wells and was repeated in three independent experiments.

### RESULTS

As shown in Figure 1, EPO treatment of osteoblasts resulted in enhanced proliferation in a dose dependent manner. Furthermore, the use of both EPO and FN together resulted in enhanced osteoblast adhesion in a dose dependent manner (Figure 2). The combination of EPO and FN showed higher adherence compared to EPO and Collagen type II (Figure 2).

### EXAMPLE 2

### Erythropoietin and fibronectin enhanced human osteoblast proliferation and adhesion MATERIALS AND EXPERIMENTAL PROCEDURES

### Cell culture

Cryopreserved secondary culture of primary human osteoblasts (HOB) was obtained from PromoCell (PromoCell GmbH, Heidelberg, Germany). The effect of erythropoietin (EPO), fibronectin (FN) and PDGF on HOB proliferation was assessed. Briefly, HOB cells were thawed gently with osteoblast growth medium (OGM, Cell System, USA) containing 10 % FBS. Cells were seeded onto 60 mm tissue culture plates at a density of 1 x 10⁷ cells/ml and cultured for 12 days in OGM supplemented with 15 % FBS, sodium penicillin G and streptomycin sulfate in a humidified incubator at 5 % CO₂-95 % nitrogen gas mixture at 37 °C. The culture medium was changed daily and the cells were allowed to replicate 3 times. On day 8, all non-adherent cells were removed with the medium exchange and the adherent cells (HOB cells) were grown for an additional period of up to 4 days. In the subsequent experiments, the beginning day of culture was defined as day 0. On the day of experiment, cell lysates were collected for protein expression determination.

### [3H]-thymidine incorporation assay

HOB cells were seeded at a density of 1 x 10⁷ cells/ml onto 6-well plates coated with or without 100 µg/ml Fibronectin (Chemicon Int.). HOB cells were grown for 5 days to reach 60-70 % confluence in OGM supplemented with 15 % FBS. HOB cells were treated with EPO (1, 5 and 10 µg/ml from Aranesp), 5 ng PDGF (Sigma) or both for 24 hours prior to experimentation. Cell cultures were placed in phenol red-free OGM (Cell-System, USA) and 0.1 % FBS. After 24 hours, the cells were placed in phenol red-free OGM containing 7.5 % dextran-charcoal stripped FBS. The cells were labeled with 10 µCi [3H]-thymidine (Shanghai, China) for the last 24 hours of culture and were rinsed with phosphate buffered saline (PBS 3 x 5 min) and 10 % trichloroacetic acid (1 x 30 min). Finally, the cells were dissolved in 0.2 ml 0.2 mol/I NaOH and left overnight at 4 °C. Radioactivity was determined by scintillation counting.

### Assaying the levels of Osteocalcin, BMP-2, BMP-4, BMP-7

Osteocalcin, BMP-2, BMP-4 and BMP-7 Quantikine ELISA kits (R&D Systems) were used to detect osteocalcin BMP-2, BMP-4 and BMP-7 levels in the culture medium of HOBs. Briefly, cells were treated with EPO, FN and PDGF (with various concentrations, as indicated above), cell culture medium was collected, placed in 96-well microtiter plates coated with monoclonal detective antibodies and incubated for 2 hours at room temperature. After removing unbound material with washing buffer (50 mM Tris, 200 mM NaCl and 0.2 % Tween 20), horseradish peroxidase conjugated streptavidin was added to bind to the antibodies. Horseradish peroxidase catalyzed the conversion of a chromogenic substrate (tetramethylbenzidine) to a colored solution, with color intensity proportional to the amount of protein present in the sample. The absorbance of each well was measured at OD 450 nm. Results were presented as the percentage of change of the activity compared to the untreated control.

### Statistical analysis

Data was expressed as means ± SEM or as percentage of control. Statistical comparison of the results was made using analysis of variance (ANOVA). Significant differences (P < 0.05) between the means of the control and test groups were analyzed by Dunnett's test. *P < 0.05, **P < 0.01, ***P < 0.001.

### RESULTS

As is evident from the results (Figure 3A), EPO (5 or 10 µg/ml) lead to a significant increase in HOB proliferation in cells not treated additionally with FN (P < 0.01, P < 0.05 respectively). In FN-coated plates (Figure 3B), EPO (1, 5 and 10 µg/ml) lead to a significant increase in HOB proliferation in a dose dependent manner (P < 0.05, P < 0.01 and P < 0.01, respectively). Culturing HOBs with PDGF (5 ng/ml) on FN-coated plates lead to an increase in HOB proliferation in a similar manner to EPO (P < 0.01). However, culturing the cells with the combination of EPO and PDGF (on FN-coated plates) had a synergistic effect, HOB proliferation increased to a level higher then either compound alone (P < 0.001) (Figure 4).

Bone morphogenic proteins (BMPs) especially 2, 4 and 7 are very important factors for the induction, differentiation and proliferation of bone tissue. Their expression in the osteoblasts plays a major role in their organization and proliferation. Therefore, BMP-2, BMP-4 and BMP-7 and osteocalcin expression were assessed in HOBs. As evident from the results (Figures 5A-B), protein levels of BMP-2, BMP-4 and BMP-7 and osteocalcin significantly increase in HOBs cultured with EPO in a dose dependent manner (Figure 5A), however, a more significant increase was recorded for cells cultured in the presence of both EPO and FN (Figure 5B).

Although the invention has been described in conjunction with specific embodiments thereof, it is evident that many alternatives, modifications and variations will be apparent to those skilled in the art.

To the extent that section headings are used, they should not be construed as necessarily limiting.

## Claims

1. A composition for use in treating a bone injury to promote bone regeneration in a subject in need thereof, wherein the composition comprises a therapeutically effective amount of Erythropoietin, Fibronectin, and platelet-derived growth factor, wherein,
(i) said therapeutically effective amount of said Erythropoietin is selected from the group consisting of:
about 1 - 50 µg/Kg for systemic administration; and
about 0.1 - 50 µg/ml for local administration, and
(ii) said therapeutically effective amount of said Fibronectin is selected from the group consisting of:
about 100 - 1000 µg/ml for systemic administration; and
about 50- 500 µg/ml for local administration, and
(iii) said therapeutically effective amount of said platelet-derived growth factor is selected from the group consisting of:
about 10 - 100 ng/ml for systemic administration; and
about 1- 50 ng/ml for local administration.

2. The composition for use according to claim 1, wherein said systemic administration is selected from the group consisting of intravenous and intrabone infusion.

3. The composition for use according to claim 1, wherein said local administration is selected from the group consisting of coated implant, coated synthetic bone and coated bone graft.

4. The composition for use according to claim 1, further comprising at least one compound capable of promoting bone regeneration.

5. The composition for use according to claim 4, wherein said at least one compound is selected from the group consisting of a bone morphogenic factor, a bone morphogenetic protein (BMP), an anti-resorptive agent, an osteogenic factor, a cartilage-derived morphogenetic protein, a parathyroid hormone, insulin-like growth factor-1 (IGF-1), fibroblast growth factor (FGF), a transforming growth factor, a noggin, an osteogenic growth peptide, a growth hormone, an estrogen, a bisphosphonate, a statin, a calcitonin, a dihydroxy vitamin D_{3'} a calcium preparation and a differentiating factor.

6. The composition for use according to claim 5, wherein said bone morphogenetic protein comprises bone morphogenetic protein 2, bone morphogenetic protein 4 and bone morphogenetic protein 7.

7. The composition for use according to claim 1, wherein said bone injury is a medical condition selected from the group consisting of osteoporosis, bone fracture or deficiency, primary or secondary hyperparathyroidism, osteoarthritis, periodontal disease or defect, an osteolytic bone disease, post-plastic surgery, post-orthopedic implantation, and post-dental implantation.

8. The composition for use according to claim 1, wherein the subject is a human being.

9. The composition for use according to claim 1, wherein said Erythropoietin, said Fibronectin, and said platelet-derived growth factor are in a co-formulation.

10. The composition for use according to claim 1, wherein said Erythropoietin, said Fibronectin, and said platelet-derived growth factor are in separate formulations.

## Patentansprüche

1. Zusammensetzung zur Verwendung bei der Behandlung einer Knochenverletzung, um eine Knochenregenerierung bei einem Patienten, der dessen bedarf, zu fördern, wobei die Zusammensetzung eine therapeutisch wirksame Menge an Erythropoietin, Fibronectin und von Platelet Derived Growth Factor umfasst, wobei
(i) die therapeutisch wirksame Menge des Erythropoietins ausgewählt ist aus der Gruppe, die aus Folgendem besteht:
etwa 1-50 µg/kg für eine systemische Verabreichung; und
etwa 0,1-50 µg/ml für eine lokale Verabreichung und
(ii) wobei die therapeutisch wirksame Menge an Fibronectin ausgewählt ist aus der Gruppe, die aus folgendem besteht:
etwa 100-1000 µg/kg für eine systemische Verabreichung; und
etwa 50-500 µg/ml für eine lokale Verabreichung und
(iii) wobei die therapeutisch wirksame Menge des Platelet Derived Growth Factor ausgewählt ist aus der Gruppe, die aus folgendem besteht:
etwa 10-100 ng/kg für eine systemische Verabreichung; und
etwa 1-50 ng/ml für eine lokale Verabreichung.

2. Zusammensetzung zur Verwendung nach Anspruch 1, wobei die systemische Verabreichung ausgewählt ist aus der Gruppe, die aus einer intravenösen und einer intraossären Infusion besteht.

3. Zusammensetzung zur Verwendung nach Anspruch 1, wobei die lokale Verabreichung ausgewählt ist aus der Gruppe, die aus einem beschichteten Implantat, einem beschichteten künstlichen Knochen und einem beschichteten Knochentransplantat besteht.

4. Zusammensetzung zur Verwendung nach Anspruch 1, ferner mindestens eine Verbindung umfassend, die in der Lage ist, eine Knochenregenerierung zu fördern.

5. Zusammensetzung zur Verwendung nach Anspruch 4 wobei die mindestens eine Verbindung ausgewählt ist aus der Gruppe bestehend aus einem Knochenmorphogenesefaktor, einem Knochenmorphogeneseprotein (BMP), einem antiresorptiven Wirkstoff, einem osteogenen Faktor, einem von Knorpel abgeleiteten Morphogeneseprotein, einem Nebenschilddrüsenhormon, insulinähnlichem Wachstumsfaktor 1 (IGF-1), Fibroblastenwachstumsfaktor (FGF), einem transformierenden Wachstumsfaktor, einem Noggin, einem das Knochenwachstum fördernden Peptid, einem Wachstumshormon, einem Östrogen, einem Bisphosphonat, einem Statin, einem Calcitonin, einem Dihydroxyvitamin D₃, einem Calciumpräparat und einem Differenzierungsfaktor.

6. Zusammensetzung zur Verwendung nach Anspruch 5, wobei das Knochenmorphogeneseprotein Knochenmorphogeneseprotein 2, Knochenmorphogeneseprotein 4 und Knochenmorphogeneseprotein 7 umfasst.

7. Zusammensetzung zur Verwendung nach Anspruch 1, wobei die Knochenverletzung ein medizinischer Zustand ist, der ausgewählt ist aus der Gruppe bestehend aus Osteoporose, Knochenbruch oder -schaden, primärem oder sekundärem Hyperparathyroeidismus, Osteoarthrits, Zahnfleischschwund oder -schädigung, einer osteolytischen Knochenkrankheit, den Folgen einer plastischen Operation, den Folgen einer orthopädischen Implantation und den Folgen einer dentalen Implantation.

8. Zusammensetzung zur Verwendung nach Anspruch 1, wobei der Patient ein Mensch ist.

9. Zusammensetzung zur Verwendung nach Anspruch 1, wobei das Erythropoietin, das Fibronectin und der Platelet Derived Growth Factor in einer gemeinsamen Formulierung vorliegen.

10. Zusammensetzung zur Verwendung nach Anspruch 1, wobei das Erythropoietin, das Fibronectin und der Platelet Derived Growth Factor in separaten Formulierungen vorliegen.

## Revendications

1. Composition pour une utilisation dans le traitement d'une lésion osseuse pour favoriser la régénération osseuse chez un sujet en ayant besoin, dans laquelle la composition comprend une quantité thérapeutiquement efficace d'érythropoiétine, de fibronectine et de facteur de croissance dérivé des plaquettes, dans laquelle,
(i) ladite quantité thérapeutiquement efficace de ladite érythropoiétine est sélectionnée à partir du groupe constitué de :
environ 1 à 50 µg/Kg pour une administration systémique ; et
environ 0,1 à 50 µg/ml pour une administration locale, et
(ii) ladite quantité thérapeutiquement efficace de ladite fibronectine est sélectionnée à partir du groupe constitué de :
environ 100 à 1000 µg/ml pour une administration systémique ; et
environ 50 à 500 µg/ml pour une administration locale, et
(iii) ladite quantité thérapeutiquement efficace dudit facteur de croissance dérivé des plaquettes est sélectionnée à partir du groupe constitué de :
environ 10 à 100 ng/ml pour une administration systémique ; et
environ 1 à 50 ng/ml pour une administration locale.

2. Composition pour une utilisation selon la revendication 1, dans laquelle ladite administration systémique est sélectionnée à partir du groupe constitué d'une perfusion intraveineuse et intraosseuse.

3. Composition pour une utilisation selon la revendication 1, dans laquelle ladite administration locale est sélectionnée à partir du groupe constitué d'un implant enduit, d'un os synthétique enduit et d'un greffon osseux enduit.

4. Composition pour une utilisation selon la revendication 1, comprenant en outre au moins un composé capable de favoriser la régénération osseuse.

5. Composition pour une utilisation selon la revendication 4, dans laquelle ledit au moins un composé est sélectionné à partir du groupe constitué d'un facteur morphogénique osseux, d'une protéine morphogénétique osseuse (BMP), d'un agent anti-résorption, d'un facteur ostéogénique, d'une protéine morphogénétique dérivée du cartilage, d'une hormone parathyroïdienne, d'un facteur de croissance insulinoïde 1 (IGF-1), d'un facteur de croissance des fibroblastes (FGF), d'un facteur de croissance de transformation, d'une noggine, d'un peptide de croissance ostéogénique, d'une hormone de croissance, d'un œstrogène, d'un bisphosphonate, d'une statine, d'une calcitonine, d'une vitamine dihydroxy D₃, d'une préparation de calcium et d'un facteur de différenciation.

6. Composition pour une utilisation selon la revendication 5, dans laquelle ladite protéine morphogénétique osseuse comprend la protéine morphogénétique osseuse 2, la protéine morphogénétique osseuse 4 et la protéine morphogénétique osseuse 7.

7. Composition pour une utilisation selon la revendication 1, dans laquelle ladite lésion osseuse est un trouble médical sélectionné à partir du groupe constitué de l'ostéoporose, d'une fracture ou déficience osseuse, de l'hyperparathyroïdisme primaire ou secondaire, de l'ostéoarthrite, d'une maladie ou anomalie périodontale, d'une maladie osseuse ostéolytique, d'une post-chirurgie plastique, d'une post-implantation orthopédique, et d'une post-implantation dentaire.

8. Composition pour une utilisation selon la revendication 1, dans laquelle le sujet est un être humain.

9. Composition pour une utilisation selon la revendication 1, dans laquelle ladite érythropoiétine, ladite fibronectine et ledit facteur de croissance dérivé des plaquettes sont dans une co-formulation.

10. Composition pour une utilisation selon la revendication 1, dans laquelle ladite érythropoiétine, ladite fibronectine et ledit facteur de croissance dérivé des plaquettes sont dans des formulations séparées.
